(19) Europäisches Patentamt
European Patent Office
Office européen des brevets

(11) Veröffentlichungsnummer: **0 252 357 B1**

# (12) EUROPÄISCHE PATENTSCHRIFT

(45) Veröffentlichungstag der Patentschrift: **01.08.90**

(51) Int. Cl.[5]: **C07C 69/88**, C07C 67/08

(21) Anmeldenummer: **87108928.0**

(22) Anmeldetag: **23.06.87**

(54) **Verfahren zur Herstellung von (4-Hydroxyphenyl-) 4-hydroxy-benzoat und seine Verwendung.**

(30) Priorität: **05.07.86 DE 3622611**

(43) Veröffentlichungstag der Anmeldung: **13.01.88 Patentblatt 88/2**

(45) Bekanntmachung des Hinweises auf die Patenterteilung: **01.08.90 Patentblatt 90/31**

(84) Benannte Vertragsstaaten: **DE FR GB IT**

(56) Entgegenhaltungen:
**EP-A- 0 139 252**
**JP-A-60 034 932**
**JP-A-60 237 048**

(73) Patentinhaber: **BAYER AG, D-5090 Leverkusen 1 Bayerwerk(DE)**

(72) Erfinder: **Müller, Hanns Peter, Dr., Im Kerberich 6, D-5068 Odenthal(DE)**
Erfinder: **Gipp, Roland, Dr., Jakob-Böhme-Strasse 2, D-5000 Köln 80(DE)**

EP 0 252 357 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es wird ein Verfahren zur Herstellung von (4-Hydroxyphenyl-)4-hydroxy-benzoat durch Veresterung von Hydrochinon und p-Hydroxybenzoesäure beschrieben, wobei erfindungsgemäß in Gegenwart eines Veresterungskatalysators in einem Reaktionsmedium gearbeitet wird, in welchem die Reaktionspartner überwiegend dispergiert vorliegen. Es wird ferner die Verwendung der nach obigen Verfahren in hohen Ausbeuten und guter technischer Reinheit zugänglichen Verbindung zur Herstellung von linearen Polycarbonaten oder Mischpolycarbonaten beansprucht.

Synthesebausteine zum Aufbau von neuartigen Polymeren, welche LC-(liquid crystal)-Bausteine in der Haupt- und/oder Seitenkette enthalten, gewinnen seit einiger Zeit zunehmend an Bedeutung.

W.R. Krigbaum et. al. beschreiben in Eur. Polym. J. 20 (3), 225 (1984) eine Studie über Polymere auf der Basis von (4-Hydroxyphenyl-)4-hydroxybenzoat. Die Autoren zeigen in dieser Arbeit, daß die Verwendung von reinen Bausteinen beim Aufbau von aromatischen Polyester das Eigenschaftsbild der resultierenden aromatischen Polyester ganz wesentlich beeinflussen.

Auch J. Jackson Jr. kommt in seiner Veröffentlichung im Brit. Polym. J. 12 (1980), 154 zu dem Schluß, daß die besten Eigenschaften von aromatischen Polyestern erhalten werden, wenn man nur symmetrisch orientierte aromatische Ringe zum Aufbau der Polyester verwendet.

Die Synthese von reinem (4-Hydroxyphenyl-)4-hydroxybenzoat (I)

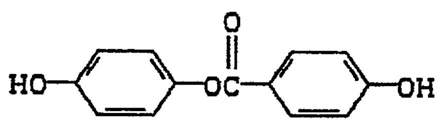

(I)

als Polymerbaustein ist aber bisher in technischem Maßstab nicht gelungen. So beschreibt Krigbaum in der oben genannten Arbeit nur eine komplizierte Synthese unter Verwendung von Schutzgruppen.

Gegenstand der vorliegenden Erfindung ist ein Verfahren zur Herstellung von (4-Hydroxyphenyl)4-hydroxybenzoat (I) durch Umsetzung von p-Hydroxybenzoesäure und Hydrochinon in etwa äquivalenten Molverhältnissen, dadurch gekennzeichnet, daß in Gegenwart eines Veresterungskatalysators in einem Reaktionsmedium gearbeitet wird, in welchem die Reaktionspartner überwiegend dispergiert vorliegen. In einer bevorzugten Ausführungsform sind die Reaktionspartner in dem Reaktionsmedium wenig und das gebildete Produkt noch weniger löslich.

Ein bevorzugter Katalysator ist die Kombination Schwefelsäure und Borsäure. Andere besonders bevorzugte Katalysatoren sind Sulfonsäuren, insbesondere aromatische Sulfonsäuren, wie z.B. p-Toluolsulfonsäure.

Gegenstand der Erfindung ist auch die Verwendung von (4-Hydroxyphenyl-)4-hydroxybenzoat (I) als Polymerbaustein zum Aufbau von linearen, aromatischen Polycarbonaten nach dem Kondensationsverfahren. Bevorzugt ist hier die Verwendung von (4-Hydroxyphenyl-)4-hydroxybenzoat, welches nach dem erfindungsgemäßen Verfahren hergestellt wird.

In einer bevorzugten Ausführungsform werden zur Durchführung des erfindungsgemäßen Verfahrens die Ausgangskomponenten 4-Hydroxybenzoesäure und Hydrochinon in einem inerten Lösemittel als Reaktionsmedium, vorzugsweise aromatischen Kohlenwasserstoff, z.B. Toluol oder Xylol, vorgelegt und mit Katalysator, z.B. mit 0,01 bis 3, vorzugsweise 0,1 bis 1,5 Gew.-% Borsäure und 0,01 bis 3, vorzugsweise 0,1 bis 1,5 Gew.-% konzentrierter Schwefelsäure versetzt und unter Rückfluß und der Entfernung des Kondensationswassers, z.B. über einen Wasserabscheider) verestert. Bevorzugt verwendet man Borsäure-/Schwefelsäuregemische mit 25 bis 70 Gew.-Teilen Borsäure und 75 bis 30 Gew.-Teilen Schwefelsäure, insbesondere etwa 40 bis 60 Gew.-Teilen Borsäure und 60 bis 40 Gew.-Teile konzentrierter Schwefelsäure. Nach 3,5 bis 4 Stunden ist die Veresterungsreaktion in der heterogenen Mischung (bei Verwendung von Xylol als Lösungsmittel) beendet. Bei niedriger siedenden Lösungsmitteln wie Toluol wird man die Veresterungszeit verlängern, bis praktisch vollständige Wasserabscheidung eingetreten ist. Man läßt den Ansatz erkalten, saugt das gut kristallisierte Rohprodukt scharf ab, wäscht mit einem inerten Lösungsmittel, vorzugsweise mit Xylol oder Toluol, dann mit verdünnter Natriumhydrogencarbonat-Lösung und anschließend mit Wasser. Nach Trocknen erhält man (4-Hydroxyphenyl-)4-hydroxy-benzoat in einer Ausbeute von 98,5 % der Theorie; Fp 242 - 245°C (bei Verwendung von Xylol als Lösungsmittel). In einer anderen bevorzugten Ausführungsform wird die Kombination Schwefelsäure/Borsäure durch p-Toluolsulfonsäure ersetzt.

Das (4-Hydroxy-phenyl-)4-hydroxybenzoat kann zur Herstellung von Polyestern mit besonderen Eigenschaften, welche auf dem flüssig-kristallinen Charakter der Baukomponente beruhen, verwendet werden. Es kann zum Aufbau von linearen Polycarbonatpolymeren verwendet werden, welche die typischen Eigenschaften von Polymeren mit flüssig-kristallinen Bausteinen besitzen. Es sind dies insbesondere erhöhte Festigkeitswerte in etwa der Orientierungsrichtung der flüssig-kristallinen Bausteine innerhalb des Polymers.

Die Verwendung von (4-Hydroxy-phenyl-)4-hydroxybenzoat (I) zur Herstellung von räumlich vernetzten Polymeren ist Gegenstand der deutschen Patentanmeldung P 36 22 613. Die erfindungsgemäß

zugängliche Verbindung (I) dient auch als Zwischenprodukt zur Herstellung von Ausgangsmaterialien mit flüssig-kristallinen Charakter, z.B. dem Bis-Epoxyd, siehe die gleichzeitig eingereichte deutsche Patentanmeldung P 36 22 610.

Beispiel 1

Herstellung von (4-Hydroxy-phenyl-)4-hydroxy-benzoat (I)

Ansatz 414 g (3 Mol) 4-Hydroxy-benzoesäure
330 g (3 Mol) Hydrochinon
1800 ml (ca. 1,56 kg) Xylol
6 g Borsäure
4,5 ml (ca. 8,2 g) konz. Schwefelsäure

In einem 3 l-Sulfierbecherglas, das mit Planschliff-Deckel, Innenthermometer, $V_2A$-Stahlrührer und - über einen Wasserabscheider - einem Rückflußkühler versehen ist und sich in einem Ölbad befindet wird das Gemisch der Reaktionskomponenten unter intensivem Rühren auf Rückfluß-Temperatur (Badtemperatur 170 - 175°C) erhitzt. Während der Umsetzung wird zu keinem Zeitpunkt eine klare Lösung beobachtet. Nach ca. 3,5-stündigem Rühren bei Rückfluß-Temperatur ist die Auskreisung des durch die Umsetzung entstandenen Wassers (ca. 55 bis 56 ml; theoretisch 54 ml) beendet. Man rührt noch 0,5 Stdn. bei Rückfluß-Temperatur nach und läßt dann - nach Möglichkeit unter weiterem Rühren - auf Raumtemperatur erkalten. Das anschließend scharf abgesaugte Rohprodukt wird mit Xylol gewaschen und bei Raumtemperatur unter vermindertem Druck getrocknet.

Das gepulverte Material wäscht man zuerst gründlich mit 3-%iger Natrium-Hydrogencarbonat-Lösung, dann mit Wasser und trocknet es bei Raumtemperatur unter vermindertem Druck.
Ausbeute: 680 g (98,5 % d. Th.)
Fp. : 242 - 245°C

Zur Abtrennung von schwer bis unlöslichen Nebenprodukten (offenbar hauptsächlich lineare Oligoester) extrahiert man I aus dem Rohprodukt erschöpfend mit heißem Aceton, fällt I aus dem auf Raumtemperatur abgekühlten Extraktionsgemisch durch Zusatz von Eiswasser unter Rühren, saugt scharf ab und trocknet über Silikagel bei Raumtemperatur und unter vermindertem Druck.
Ausbeute: 563 g (81,6 % d. Th.).

Umkristallisation aus Glykolmonomethyletheracetat[1)] und Trocknung wie oben liefern 492 g (71,4 %)[2)] I vom Fp.: 241-243°C, das eine für weitere Umsetzungen ausreichende Reinheit besitzt.

[1)] Erforderlichenfalls unter Zusatz von A-Kohle.

[2)] Verwendet man statt Glykolmonomethyletheracetat die Mutterlauge der Umkristallisation des Produktes eines vorherigen Ansatzes, so erhält man I von gleicher Reinheit mit einer Ausbeute von ca. 102 % d.Th., d.h. die Gesamtausbeute aus beiden Ansätzen beträgt dann fast 87 % d.Th.

Beispiel 2

Herstellung von (4-Hydroxy-phenyl-)4-hydroxy-benzoat

Ansatz 414 g (3 Mol) 4-Hydroxy-benzoesäure
330 g (3 Mol) Hydrochinon
2500 ml (ca. 2,17 kg) Xylol
10 g p-Toluolsulfonsäure

Die Umsetzung wird gemäß Beispiel 1 aber in einem 4 l-Sulfierbecherglas vorgenommen.
Ausbeute: 640 g (92,7 % d.Th.)
Fp.: 242 - 245°C

Es entsteht oligomerenfreies Material, das bei Raumtemperatur in Aceton vollständig löslich ist.

**Patentansprüche**

1. Verfahren zur Herstellung von (4-Hydroxyphenyl-)4-hydroxybenzoat durch Umsetzung von p-Hydroxybenzoesäure und Hydrochinon in etwa äquivalenten Molverhältnissen, dadurch gekennzeichnet, daß in Gegenwart eines Veresterungskatalysators in einem Reaktionsmedium gearbeitet wird, in welchem die Reaktionspartner überwiegend dispergiert vorliegen.

2. Verfahren nach Anspruch 1, dadurch gekennzeichnet, daß man als Katalysator 0,01 - 3 Gew.-% Borsäure und 0,01 - 3 Gew.-% konzentrierte Schwefelsäure, bezogen auf den Feststoffanteil der Ausgangsmaterialien, verwendet.

3. Verfahren nach Ansprüchen 1 und 2, dadurch gekennzeichnet, daß man als Katalysator Borsäure-/Schwefelsäuregemische mit 25 - 70 Gew.-Teilen Borsäure und 75 -30 Gew.-Teilen Schwefelsäure verwendet.

4. Verfahren nach wenigstens einem der vorhergehenden Ansprüche, dadurch gekennzeichnet, daß als Katalysator eine Sulfonsäure verwendet wird.

5. Verfahren nach wenigstens einem der vorhergehenden Ansprüche, dadurch gekennzeichnet, daß als Katalysator p-Toluolsulfonsäure verwendet wird.

6. Verfahren nach wenigstens einem der vorhergehenden Ansprüche, dadurch gekennzeichnet, daß man aromatische Kohlenwasserstoffe als Reaktionsmedium verwendet und die Veresterungsreaktion unter Wasserabscheidung am Rückfluß durchführt.

**Claims**

1. A process for the production of (4-hydroxyphenyl)-4-hydroxybenyzoate by reaction of p-hydroxybenzoic acid and hydroquinone in substantially equivalent molar ratios, characterized in that the reaction is carried out in the presence of an esterification catalyst in a reaction medium in which the reactants are present predominantly in dispersion.

2. A process as claimed in claim 1, characterized in that 0.01 to 3% by weight boric acid and 0.01 to 3% by weight concentrated sulfuric acid, based on the solids content of the starting materials, are used as catalyst.

3. A process as claimed in claims 1 and 2, characterized in that mixtures of boric acid and sulfuric acid containing 25 to 70 parts by weight boric acid and 75 to 30 parts by weight sulfuric acid are used as catalyst.

4. A process as claimed in at least one of the preceding claims, characterized in that a sulfonic acid is used as catalyst.

5. A process as claimed in at least one of the preceding claims, characterized in that p-toluenesulfonic acid is used as catalyst.

6. A process as claimed in at least one of the preceding claims, characterized in that aromatic hydrocarbons are used as the reaction medium and the esterification reaction is carried out under reflux conditions with separation of the water formed.

**Revendications**

1. Procédé de production de 4-hydroxybenzoate de 4-hydroxyphényle par réaction de l'acide p-hydroxybenzoïque et d'hydroquinone en quantités molaires à peu près équivalentes, caractérisé en ce qu'on opère en présence d'un catalyseur d'estérification dans un milieu de réaction dans lequel les partenaires réactionnels sont présents principalement à l'état dispersé.

2. Procédé suivant la revendication 1, caractérisé en ce qu'on utilise comme catalyseur 0,01 à 3% en poids d'acide borique et 0,01 à 3% en poids d'acide sulfurique concentré, par rapport à la proportion de substance solide des matières de départ.

3. Procédé suivant les revendications 1 et 2, caractérisé en ce qu'on utilise comme catalyseur des mélanges d'acide borique et d'acide sulfurique contenant 25 à 70 parties en poids d'acide borique et 75 à 30 parties en poids d'acide sulfurique.

4. Procédé suivant au moins l'une des revendications précédentes, caractérisé en ce qu'on utilise comme catalyseur un acide sulfonique.

5. Procédé suivant au moins l'une des revendications précédentes, caractérisé en ce qu'on utilise comme catalyseur l'acide p-toluènesulfonique.

6. Procédé suivant au moins l'une des revendications précédentes, caractérisé en ce qu'on utilise des hydrocarbures aromatiques comme milieu réactionnel et on conduit la réaction d'estérification au reflux, avec séparation d'eau.